# EUROPÄISCHE PATENTSCHRIFT

(11) **EP 0 484 331 B1**
(45) Veröffentlichungstag und Bekanntmachung des Hinweises auf die Patenterteilung: **19.01.1994**
(21) Anmeldenummer: 90905492.6
(22) Anmeldetag: 04.04.1990
(51) Int. Cl.: A61K 9/16, C30B 29/54, C07C 217/36

(54) **EINKRISTALLE VON $g(b)-PHENYLPROPIOPHENONEN**
MONOCRYSTALS OF $g(b)-PHENYLPROPIOPHENONES
MONOCRISTAUX DE $g(b)-PHENYLPROPIOPHENONES

(30) Priorität: 06.04.1989 DE 3911069
(43) Veröffentlichungstag der Anmeldung: 13.05.1992
(73) Patentinhaber: KNOLL AG, D-67061 Ludwigshafen (DE)
(72) Erfinder: FRICKE, Helmut, D-6704 Mutterstadt (DE); MOEST, Thomas, D-2082 Moorrege (DE); BUEHLER, Volker, D-7500 Karlsruhe (DE); MUELLER-PELTZER, Herbert, D-6900 Heidelberg (DE)
(74) Vertreter: Karau, Wolfgang, Dr.
(86) Internationale Anmeldenummer: EP9000529
(87) Internationale Veröffentlichungsnummer: WO9011755

(56) Entgegenhaltungen:
- EP-A- 0 119 480
- WO-A-83/00688
- DE-A- 2 001 431

## Beschreibung

Die vorliegende Erfindung betrifft Einkristalle von β-Phenylpropiophenonen sowie deren Verwendung zur Herstellung von Depotformen.

Propafenon (I, R = -CH₂-CH₂-CH₃) ist ein pharmazeutischer Wirkstoff, der gegen Arrythmien eingesetzt wird. Obwohl dieser Wirkstoff schon längere Zeit verwendet wird, existiert bislang keine Depot-Arzneiform mit diesem Wirkstoff. Auch für die entsprechende 1,1-Dimethylpropyl-Verbindung existiert bislang keine Depotform.

Aus der Pharmazeutischen-Zeitung Nr. 48, Seite 9 (01.12.88) ist es bekannt, daß man durch Bildung von Makrokristallen eine langsame Wirkstoffauflösung erreichen kann. Wie aber am Beispiel des Nitrofurantoins gezeigt wurde, läßt sich durch die Applikation von Kristallen lediglich das Auftreten einer Konzentrationsspitze verhindern, nicht aber eine Depotform erhalten, die einen deutlich späteren maximalen Blutspiegel hervorruft.

Es wurde nun gefunden, daß sich bestimmte Einkristalle von β-Phenylpropiophenonen gut zur Herstellung von Depotpräparaten mit wunschgemäßen Blutspiegeln eignen.

Gegenstand der Erfindung sind Einkristalle von β-Phenylpropiophenonen der Formel I 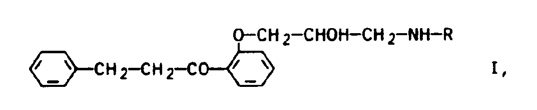
worin R der Rest -CH₂-CH₂-CH₃ oder -C(CH₃)₂-CH₂-CH₃ ist, deren Volumen im Bereich von 0,004 bis 1,2 mm³ liegt. Vorzugsweise haben die Einkristalle ein Volumen von 0,1 bis 0,6 mm³.

Gegenstand der Erfindung ist weiter die Verwendung der oben genannten Einkristalle zur Herstellung von Depotpräparaten.

Die Einkristalle der Verbindungen I lassen sich nach bekannten Methoden herstellen. Bevorzugt sind sphärische Kristalle, weil sich solche Kristalle besser für die Verarbeitung zu abgeteilten Arzneiformen eignen. Man erhält solche Kristalle nach den in der EP-OS 119 480 angegebenen Verfahren.

Zur Herstellung einer Applikationsform werden die Einkristalle vorzugsweise in eine Kapsel gefüllt. Man kann die Einkristalle aber auch in üblicher Weise zu Dragees oder Tabletten verarbeiten.

Mit Hilfe der Einkristalle ist es möglich, den Plasmaspiegel über einen längeren Zeitraum praktisch konstant zu halten, obwohl die Substanzen nur 2 x täglich appliziert werden. Das gilt insbesondere für das Propafenon. Der Retardeffekt beruht ausschließlich auf dem physikalischen Vorgang des zeitabhängigen Auflösens der Kristalle.

### Beispiel 1

In einem handelsüblichen 50-l-Rührkessel mit Schrägblattrührer, Doppelmantel und Stromstörer wurden 40 l einer auf 65°C erwärmten, gesättigten Lösung von Propafenonhydrochlorid in Methanol intensiv gerührt.

Nach Zugabe von 350 g Propafenonhydrochlorid-Kristallkeimen mit einer Korngröße von 100 - 200 µm ließ man langsam mit einer Kühlrate von 4 K/h unter stetigem Rühren mit einer Drehzahl von n = 100 min⁻¹ auf 20°C abkühlen.

Das entstandene Kristallisat wurde in wenig kaltem Methanol aufgenommen, sofort über eine Nutsche abgesaugt, mit wenig kaltem Methanol nachgewaschen und im Vakuumtrockenschrank bei 50°C und 250 - 400 mbar getrocknet.

Das Produkt bestand aus farblosen sphärischen Kristallen mit einem Korngrößenbereich von 0,4 - 1,2 mm, wobei ein ausgeprägtes Maximum der Häufigkeitsverteilung im Bereich 0,6 - 0,8 mm, entsprechend einem Kornvolumenbereich von 0,11 - 0,27 mm³, lag.

### Beispiel 2

In der Apparatur gemäß Beispiel 1 wurde die gesättigte auf 65°C erwärmte methanolische Propafenonhydrochlorid-Lösung mit einer Kühlrate von 10 K/h unter stetigem Rühren mit einer Drehzahl von n = 100 min⁻¹ ohne Impfkristallzugabe auf 20°C abgekühlt. Das entstandene Kristallisat wurde gemäß Beispiel 1 abgetrennt und getrocknet.

Das Produkt bestand aus farblosen, mikroskopisch erkennbar gerundeten Kristallen mit einem Korngrößenbereich von 100 - 600 µm, wobei ein Maximum der Häufigkeitsverteilung im Bereich von 0,2 - 0,4 mm, entsprechend einem Kornvolumenbereich von 0,004 - 0,034 mm³, lag.

### Beispiel 3

In einem handelsüblichen 50-l-Rührkessel mit Schrägblattrührer und Doppelmantel wurden 40 l einer auf 65°C erwärmten, gesättigten Lösung von Propafenonhydrochlorid in Methanol intensiv gerührt und mit einer Abkühlrate von 20 K/h unter stetigem Rühren mit einer Drehzahl von n = 60 min⁻¹ auf 20°C abgekühlt. Das entstandene Kristallisat wurde gemäß Beispiel 1 abgetrennt und getrocknet.

Das Produkt bestand aus farblosen bis weißlich schimmernden kantigen Plättchen mit einer Korngrößenverteilung von 0,1 - 2,0 mm mit einem breiten Maximum der Häufigkeitsverteilung im Bereich 0,5 - 1,2 mm.

## Patentansprüche

1. Einkristalle von β-Phenylpropiophenonen der Formel I 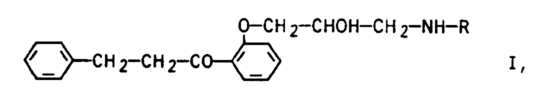 worin R der Rest -CH₂-CH₂-CH₃ oder -C(CH₃)₂-CH₂-CH₃ ist, deren Volumen im Bereich von 0,004 bis 1,2 mm³ liegt.

2. Einkristalle des Propafenons gemäß Anspruch 1.

3. Einkristalle gemäß Anspruch 1, deren Volumen im Bereich von 0,1 bis 0,6 mm³ liegt.

4. Verwendung der Einkristalle gemäß Anspruch 1 zur Herstellung von Depotpräparaten.

5. Verfahren zur Herstellung von Depotpräparaten, wobei man Einkristalle von β-Phenylpropiophenonen der Formel I nach Anspruch 1 in Kapseln füllt oder in üblicher Weise zu Dragees oder Tabletten verarbeitet.

6. Verfahren zur Herstellung von Depotpräparaten, wobei man Einkristalle von Propafenon nach Anspruch 1 in Kapseln füllt oder in üblicher Weise zu Dragees oder Tabletten verarbeitet.

## Claims

1. Single crystals of β-phenylpropiophenones of the formula I 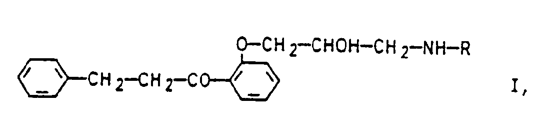 in which R is the radical -CH₂-CH₂-CH₃ or -C(CH₃)₂-CH₂-CH₃, whose volume is in the range of from 0.004 to 1.2 mm³.

2. Single crystals of propafenone as claimed in claim 1.

3. Single crystals as claimed in claim 1, whose volume is in the range of from 0.1 to 0.6 mm³.

4. The use of single crystals as claimed in claim 1 for preparing depot products.

5. A process for preparing depot products, wherein single crystals of β-phenylpropiophenones of the formula I as claimed in claim 1 are packed into capsules or processed in a customary manner to give coated tablets or tablets.

6. A process for preparing depot products, wherein single crystals of propafenone as claimed in claim 1 are packed into capsules or processed in a customary manner to give coated tablets or tablets.

## Revendications

1. Monocristaux de β-phénylpropiophénones de formule I 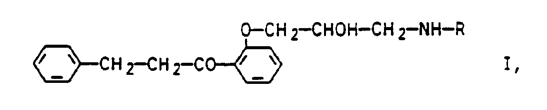 dans laquelle R représente un groupe -CH₂-CH₂-CH₃ ou -C(CH₃)₂-CH₂-CH₃, dont le volume se situe dans l'intervalle de 0,004 à 1,2 mm3.

2. Monocristaux de "Propafenon" selon la revendication 1.

3. Monocristaux selon la revendication 1, dont le volume se situe dans l'intervalle de 0,1 à 0,6 mm3.

4. Utilisation des monocristaux selon la revendication 1 pour la préparation de compositions-retards.

5. Procédé pour la préparation de compositions-retards, dans lequel on introduit des monocristaux de β-phénylpropiophénones de formule I de la revendication 1 dans des capsules ou on les met de la manière habituelle à l'état de dragées ou de comprimés.

6. Procédé de préparation de compositions-retards, dans lequel on introduit des monocristaux de Propafenon selon la revendication 1 dans des capsules ou on les met de la manièe habituelle sous la forme de dragées ou de comprimés.
